(19) Europäisches Patentamt
European Patent Office
Office européen des brevets

(11) Veröffentlichungsnummer : **0 002 476**
**B1**

# (12) EUROPÄISCHE PATENTSCHRIFT

(45) Veröffentlichungstag der Patentschrift : **11.02.81**

(51) Int. Cl.[3] : **G 03 C 1/34**, G 03 C 7/30, C 07 C 33/40

(21) Anmeldenummer : **78101533.4**

(22) Anmeldetag : **04.12.78**

## (54) Fotografisches Material und fotografische Verarbeitungsbäder mit einem Stabilisierungsmittel sowie ein Verfahren zur Herstellung fotografischer Bilder in Gegenwart eines Stabilisierungsmittels.

(30) Priorität : **15.12.77 DE 2756030**

(43) Veröffentlichungstag der Anmeldung : **27.06.79 (Patentblatt 79/13)**

(45) Bekanntmachung des Hinweises auf die Patenterteilung : **11.02.81 Patentblatt 81/06**

(84) Benannte Vertragsstaaten : **BE DE FR GB**

(56) Entgegenhaltungen :
**DE - A - 2 648 839**
**DE - B - 1 149 246**
**FR - A - 2 198 169**
**FR - A - 2 215 641**
**US - A - 2 989 568**
**US - A - 3 212 900**
**US - A - 3 305 376**
**E.J. BIRR « Stabilization of photographic silver halide emulsions », 1974, The focal press, London, Seiten 35, 36, 43, 44, 55-59**
**PHOTOGRAPHIC ABSTRACTS, Vol. 48, Nr. 5, 1968, London, « Effects of acetylene derivatives on photographic emulsions », Seite 169, Zusammenfassung Nr. 1580**

(73) Patentinhaber : **AGFA-GEVAERT Aktiengesellschaft Patentabteilung**
**D-5090 Leverkusen 1 (DE)**

(72) Erfinder : **Jäger, Gerhard, Dr.**
**Claudiusweg 5**
**D-5600 Wuppertal 1 (DE)**
Erfinder : **von König, Anita, Dr.**
**Schoenwasserstrasse 230**
**D-4150 Krefeld (DE)**
Erfinder : **Liebe, Werner, Dr.**
**Am Thelenhof 28**
**D-5090 Leverkusen (DE)**
Erfinder : **Voigt, Armin, Dr.**
**An der Ruthen 6**
**D-5000 Köln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 002 476 B1

# Fotografisches Material und fotografische Verarbeitungsbäder mit einem Stabilisierungsmittel sowie ein Verfahren zur Herstellung fotografischer Bilder in Gegenwart eines Stabilisierungsmittels

Die Erfindung betrifft ein fotografisches Material mit mindestens einer Silberhalogenidemulsionsschicht, welches durch den Zusatz von Stabilisierungsmitteln, die geeignet sind, das fotografische Material gegen die Bildung von Schleier, Farbschleier und gegen eine Verflachung der Gradation zu stabilisieren, verbessert ist.

Die Erfindung betrifft weiter fotografische Verarbeitungsbäder mit einem Stabilisierungsmittel sowie ein Verfahren zur Herstellung fotografischer Bilder in Gegenwart eines Stabilisierungsmittels.

Materialien mit lichtempfindlichen Silberhalogenidemulsionen, insbesondere chemisch sensibilisierte, neigen bekanntlich zur Bildung von Schleiern, hervorgerufen durch Keime, die ohne Belichtung entwickelbar sind. Diese Schleierbildung tritt insbesondere auf bei zu langer Lagerung, besonders bei erhöhter Temperatur und Luftfeuchtigkeit, bei zu langer Entwicklung oder Entwicklung bei zu hohen Temperaturen durch bestimmte Zusätze und bei stark gereiften Emulsionen.

Es ist bekannt, fotografischen Silberhalogenidemulsionen zur Verminderung dieser Schleierbildung sogenannte Antischleiermittel oder Stabilisierungsmittel zuzusetzen. Stabilisierende Wirkung besitzen z.B. heterocyclische Mercaptoverbindungen, z.B. solche, die in DT-AS 1 183 371, DT-OS 2 308 530 und DT-OS 1 622-271 beschrieben sind.

Diesen Stabilisierungsmitteln haftet jedoch als Nachteil an, daß sie in wirksamen Konzentrationen im allgemeinen die Empfindlichkeit der stabilisierten Emulsion herabsetzen, wodurch deren Anwendbarkeit beeinträchtigt wird. Auch die Gradation der Emulsion kann durch diese Stabilisatoren ungünstig beeinflußt werden.

An Stabilisierungsmittel werden, insbesondere auch in Bezug auf die Wechselwirkung mit anderen fotografischen Zusätzen und im Hinblick auf die Vielfältigkeit fotografischer Reproduktionsprozesse und der hierfür verwendeten fotografischen Materialien die verschiedensten Anforderungen gestellt, denen die bekannten Stabilisierungsmittel nicht genügen.

Es ist weiterhin bekannt, daß die mit den üblichen silberhalogenidhaltigen Materialien hergestellten farbigen Bilder oftmals Farbschleier oder Verfärbungen zeigen. Die Bildung eines Farbschleiers ist darauf zurückzuführen, daß die Entwicklerverbindungen unter der Einwirkung von Luft bis zu einem gewissen Grade oxidierbar sind und daß die so oxidierten Entwicklerverbindungen mit dem Farbkuppler auch an solchen Stellen in dem fotografischen Aufzeichnungsmaterial kuppeln, in denen ursprünglich kein Silberbild erzeugt worden ist. Diese störende Oxidation des Entwicklers kann außer durch Einwirkung von Luft durch Zusätze erfolgen, die in den Emulsionen vorhanden sind. Farbschleier und Verfärbungen sind insbesondere in solchen Aufzeichnungsmaterialien festzustellen, die in den lichtempfindlichen Schichten eingearbeitete Kuppler enthalten. Der Farbschleier und die Verfärbungen können im allgemeinen nicht nach den Verfahren verhindert werden, die zur Stabilisierung gegen Silberschleier üblich sind.

Zur Stabilisierung gegen Farbschleier in farbfotografischen Materialien ist die Verwendung von Alkyl- und Dialkyl-hydrochinon-Derivaten in den US-Patentschriften 2 403 721 und 2 701 197 und der deutschen Offenlegungsschrift 2 110 521 vorgeschlagen worden.

Nachteilig an diesen Verbindungen ist jedoch, daß viele von ihnen unter Anwendung von 2- bis 4-stufigen Verfahren nur schwierig herzustellen sind und daß sie zum Teil nicht genügend diffusionsfest sind, so daß sie in mehrschichtigen fotografischen Aufzeichnungsmaterialien zwischen den einzelnen Schichten wandern können, wodurch unerwünschte Nebenwirkungen erzielt werden. Zum Teil kristallisieren diese Verbindungen während oder nach ihrer Aufbringung aus, oder beeinflussen in ungünstiger Weise die physikalischen oder chemischen Eigenschaften der Schichten, wobei insbesondere nachteilig ist, daß einige Alkylhydrochinone während der Beschichtung oder der Entwicklung durch eine Oxidationsreaktion gefärbte Nebenprodukte ergeben. Diese störenden Nebenprodukte verfärben das fotografische Material, was insbesondere bei Kopiermaterialien störend ist.

Es ist ferner bekannt, eine Verbesserung der Farbwiedergabe dadurch zu erreichen, daß zwischen der lichtempfindlichen Silberhalogenidemulsionsschicht und einer die Farbkuppler enthaltenden Schicht eine Zwischenschicht angeordnet wird, die so aufgebaut ist, daß die Diffusion der Oxidationsprodukte der Entwickler in die farbkupplerhaltige Schicht unterdrückt wird. Für diesen Zweck werden dieser Schicht u.a. solche Verbindungen einverleibt, die mit den Entwickleroxidationsprodukten unter Bildung farbloser Verbindungen reagieren. Der Farbschleier wird aber durch diese sogenannten « Weißkuppler » in für die Praxis nicht ausreichendem Maße vermindert.

Es ist weiterhin gemäß der deutschen Patentschrift 2 304 321 bekannt, farbfotografisches Material in Gegenwart von 2-Propinylthioäther-Derivaten zur Verminderung von Verfärbungen zu verarbeiten. Aber auch diese Maßnahme wird den gestiegenen Anforderungen, insbesondere bei Hochtemperaturverarbeitung, nicht mehr gerecht.

Schließlich ist es bekannt, daß bei fotografischen Materialien — insbesondere bei Materialien mit relativ steiler Gradation — bei Lagerung eine Verflachung der Gradation auftreten kann. Aus der US-PS 3 488 709 ist es bekannt, die Gradation von Emulsionen, die Rhodiumsalze zur Gradationaufsteilung enthalten, durch Zugabe von Cadmiumsalzen zu stabilisieren. Man ist aber in zunehmendem Maße bestrebt, die Verwendung von Cadmiumsalzen in fotografischen Materialien zu vermeiden.

Es besteht also weiterhin ein Bedarf an Stabilisierungsmitteln, die fotografische Materialien gegen

Schleier, Farbschleier und Gradationsverflachung stabilisieren.

Der Erfindung lag die Aufgabe zugrunde, Stabilisierungsmittel aufzufinden, die fotografische Materialien gegen Schleier, Farbschleier und Gradationsverflachung stabilisieren können. Eine weitere Aufgabe ist die Bereitstellung von fotografischen Materialien mit mindestens einer Silberhalogenidemulsionsschicht, die mit diesen Verbindungen stabilisiert sind.

Gegenstand der Erfindung ist ein fotografisches Material mit wenigstens einer Silberhalogenidemulsionsschicht und wenigstens einem Stabilisierungsmittel gemäß folgender Formel I

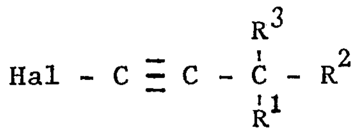

I

worin bedeuten

$R^1$ Alkyl, insbesondere Alkyl mit 1-4 C-Atomen Aryl, insbesondere Phenyl, welches ggf. substituiert sein kann, vorzugsweise mit Halogen oder einer Nitro-, Phenyl-, Phenoxy-, Alkyl-, $CF_3O$- oder $CF_3$-Gruppe ;
Aralkyl ; ein heterocyclischer Rest, beispielsweise Pyridyl, insbesondere Pyridyl-(4), Pyridyl-(3) und Pyridyl-(2) ;

$R^2$ OH oder ein stickstoffhaltiger heterocyclischer Rest mit 5 Ringatomen, beispielsweise 1-Imidazolyl ;

$R^3$ Phenyl, ggf. ein- oder mehrfach substituiert, insbesondere mit Alkyl mit 1-4 C-Atomen, Halogen oder einer Nitrogruppe ;

Hal Br, J ;

oder dessen Salz

Gegenstand der Erfindung ist weiterhin ein Verfahren zur Herstellung fotografischer Bilder durch Verarbeitung fotografischer Aufzeichnungsmaterialien in Gegenwart der erfindungsgemäß zu verwendenden Verbindungen.

Die genannten Alkyl-, Aryl- und heterocyclischen Reste können weitere Substituenten enthalten, wobei diese Substituenten ihrerseits substituiert sein können.

Unter einem Salz der Verbindungen der Formel I werden insbesondere diejenigen Verbindungen verstanden, in denen der Rest $R^2$ einen Rest folgender allgemeinen Formel darstellt und in der $X^-$ das zugehörige Anion ist :

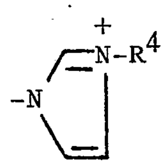

$X^-$

worin

$R^4$ ein Alkyl- bzw. Aralkylrest (insbesondere Benzyl- und am Aromaten durch Alkyl $C_1$-$C_4$ und Halogen substituierter Benzylrest), Wasserstoff und

X Halogen (insbesondere Chlor, Brom, Jod) oder ein Alkyl- bzw. Arylsulfatanion, insbesondere ein Tosyl- oder Mesylrest ist.

Die folgenden Verbindungen der Formeln II bis IV haben sich als besonders geeignet erwiesen :

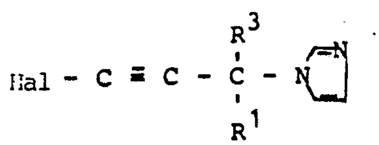

II

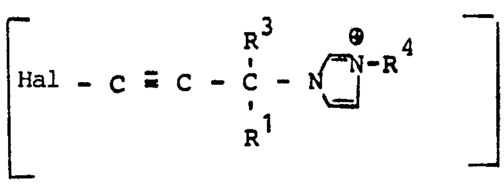

$X^-$ III

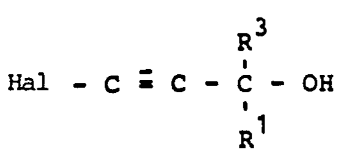

IV

worin
R[1] und Hal die unter Formel I aufgeführte Bedeutung haben,
R[3] ein gegebenenfalls einfach oder mehrfach substituierter Phenylrest ist und
R[4] und X die oben angegebene Bedeutung haben.

Beispiele für erfindungsgemäß verwendbare Verbindungen sind in den folgenden Tabellen I und II enthalten :

TABELLE I

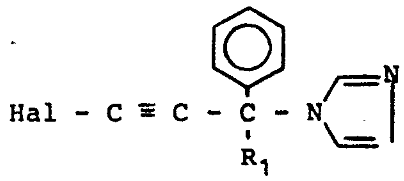

| Verbindung-Nr. | Hal | $R^1$ | Schmelzpunkt |
|---|---|---|---|
| 1 | Br | Phenyl | 193° |
| 2 | J | Phenyl | Zers. 199° |
| 3 | J | Isopropyl | 159-160° |
| 4 | J | m-Tolyl | Zers. 168-169° |
| 5 | Br | m-Tolyl | 144-145,5° |

TABELLE II

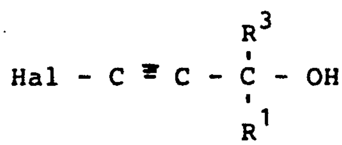

| Verbindung Nr. | Hal | $R^1$ | $R^3$ | Schmelz-punkt oder Brechungsindex |
|---|---|---|---|---|
| 6 | Br | Phenyl | Phenyl | Fp : 60-62° |
| 7 | J | Phenyl | Phenyl | Fp : 106-07° |
| 8 | J | Phenyl | p-Nitrophenyl | Fp : 116-18° |
| 9 | J | Phenyl | o-Fluorphenyl | Fp : 81-82° |
| 10 | Br | Phenyl | o-Fluorphenyl | $n_D^{20}$ 1.6040 |
| 11 | Br | 3-Pyridyl | Phenyl | Fp : 107-08° |
| 12 | J | 4-Pyridyl | Phenyl | Fp : Zers. 176° |
| 13 | Br | 4-Pyridyl | Phenyl | Fp : 166-67° |
| 14 | J | 2-Pyridyl | Phenyl | Fp : 86-87° |
| 15 | J | 4-Pyridyl | p-Tolyl | Fp : Zers. 179-80° |
| 16 | Br | 4-Pyridyl | p-Tolyl | Fp : Zers. 189° |
| 17 + 1 Mol Pyridin | Br | p-Biphenylyl | o-Chlorphenyl | Fp : 77-79° |
| 18 | J | p-Biphenylyl | o-Chlorphenyl | harzig |
| 19 | Br | p-Biphenylyl | m-Chlorphenyl | Fp : 106-07° |
| 20 | J | p-Biphenylyl | m-Chlorphenyl | Fp : 99-100° |
| 21 | Br | p-Biphenylyl | m-Tolyl | Fp : 131-32° |
| 22 | J | p-Biphenylyl | m-Tolyl | Fp : 137-39° |
| 23 | Br | p-Phenoxyphenyl | o-Chlorphenyl | Fp : 95-96° |
| 24 | J | p-Phenoxyphenyl | o-Chlorphenyl | Fp : 104-05° |
| 25 | J | p-Phenoxyphenyl | m-Chlorphenyl | Öl $n_D^{20}$ 1.6272 |
| 26 | J | p-Phenoxyphenyl | m-Tolyl | Fp : 82-83° |
| 27 | Br | p-Phenoxyphenyl | m-Tolyl | Öl $n_D^{40}$ 1.6172 |
| 28 | J | Phenyl | p-Trifluormethoxyphenyl | Öl $n_D^{20}$ 1.5697 |
| 29 | Br | Phenyl | p-Trifluormethoxyphenyl | Öl $n_D^{20}$ 1.5427 |
| 30 | J | Phenyl | 3-Chlor-4-trifluormethyl-phenyl | Öl $n_D^{40}$ 1.5860 |

| | | | | |
|---|---|---|---|---|
| 31 | Br | Phenyl | 3-Chlor-4-trifluormethyl-phenyl | Öl $n_D^{40}$ 1.5683 |
| 32 | J | Phenyl | 2,6-Difluorphenyl | Fp : 93-95° |
| 33 | Br | Phenyl | 2,6-Difluorphenyl | Fp : 60-61° |
| 34 | J | 3-Pyridyl | p-Tolyl | Fp : 148-50° |
| 35 | Br | 3-Pyridyl | p-Tolyl | Fp : Zers. 152° |
| 36 | J | Phenyl | 2,4-Difluorphenyl | Öl $n_D^{20}$ 1.6098 |
| 37 | Br | Phenyl | 2,4-Difluorphenyl | Öl $n_D^{20}$ 1.5845 |

Beispielhaft für Salze von Verbindungen gemäß Formel I seien die folgenden Quartärsalze der Verbindung 2 aufgeführt :

2 a)

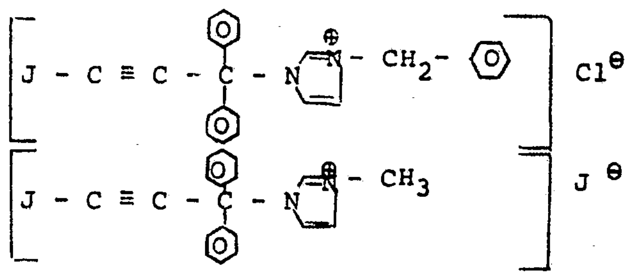

2 b)

Die in der erfindungsgemäßen Weise zu verarbeitenden Verbindungen können nach prinzipiell bekannten Methoden hergestellt werden durch Umsetzung der entsprechenden Propine mit Halogen. Beispielsweise ist die Herstellung einiger der in den Tabellen angegebenen Verbindungen im folgenden beschrieben :

Herstellung der Verbindung Nr. 2

(3-Jod-1.1-diphenyl-1-(imidazolyl-1)-propin)

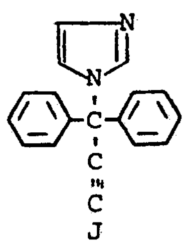

Zu 25,8 g (0,1 Mol) 1.1-Diphenyl-1-(imidazolyl-1)-propin in 200 ml Methanol werden bei 20-30 °C 44,5 ml 45 %ige Natronlauge zugetropft und gleichzeitig 25,4 g (0,1 Mol) Jod portionsweise eingetragen. Nach einer Stunde wird das Reaktionsgemisch mit 1000 ml Wasser versetzt. Die ausgeschiedenen Kristalle werden abgesaugt, zunächst mit Wasser und dann mit heißem Acetonitril gewaschen. Man erhält so 26,8 g (= 69,8 % der Theorie) 3-Jod-1.1-diphenyl-1-(imidazolyl-1)-propin vom Schmelzpunkt 199 °C (Zersetzung).

Herstellung der Verbindung Nr. 1

3-Brom-1.1-diphenyl-1-(imidazolyl-1)-propin

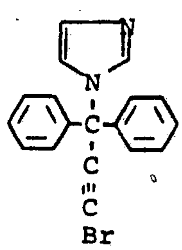

5,5 ml (0,11 Mol) Brom werden bei 0-5 °C zu 22 ml konzentrierter Natronlauge in 50 ml Wasser zugetropft. In diese Lösung werden 25,8 g (0,1 Mol) 1.1-Diphenyl-1-(imidazolyl-1)-propin, gelöst in 100 ml Pyridin, allmählich eingerührt. Dabei steigt die Innentemperatur auf 40 °C an. Man läßt noch drei Stunden bei Raumtemperatur nachrühren, versetzt mit 1000 ml Wasser, saugt das ausgeschiedene Kristallisat ab

und wäscht mit Acetonitril und Äther nach. Man erhält so 30,7 g (= 91 % der Theorie) 3-Brom-1.1-diphenyl-1-(imidazolyl-1)-propin vom Schmelzpunkt 193 °C.

Herstellung der Verbindung Nr. 7

(3-Jod-1.1-diphenyl-propin-2-ol)

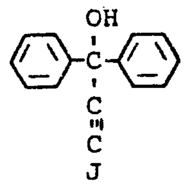

Zu einer Lösung von 41,6 g (0,2 Mol) 1.1-Diphenyl-propin-2-ol in 500 ml Methanol werden unter Kühlung bei 20 °C gleichzeitig 80 ml konzentrierte Natronlauge zugetropft und 50,8 g (0,2 Mol) Jod portionsweise eingetragen. Man läßt drei Stunden bei Raumtemperatur rühren, filtriert und versetzt das Filtrat portionsweise mit 1000 ml Wasser. Der ausgeschiedene kristalline, farblose Niederschlag wird abgesaugt, mit Wasser gewaschen und getrocknet. Man erhält so 53,5 g (= 80 % der Theorie) 3-Jod-1.1-diphenyl-propin-2-ol vom Schmelzpunkt 106-107 °C.

Herstellung der Verbindung Nr. 21

(3-Brom-1-(3-tolyl)-1-(4-biphenylyl)-propin-2-ol)

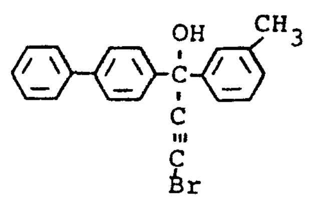

Zu 129 ml 10 %ige Natronlauge werden unter Rühren bei 0-5 °C 8,2 ml (0,16 Mol) Brom zugetropft. In diese Lösung werden bei 20 °C 41,8 g (0,14 Mol) 1-(3-Tolyl)-1-(4-biphenylyl)-propin-2-ol, gelöst in 150 ml Pyridin, eingerührt. Nach drei Stunden wird in 1000 ml Wasser gegossen und das abgeschiedene Öl in Essigester aufgenommen. Die organische Phase wird mehrmals mit Wasser ausgeschüttelt, über Natriumsulfat getrocknet, filtriert und unter vermindertem Druck eingedampft. Das zurückbleibende Öl wird in wenig Äther aufgenommen und portionsweise mit Petroläther versetzt. Die ausgeschiedenen farblosen Kristalle werden abgenutscht und getrocknet. Man erhält so 34,8 g (= 65,9 % der Theorie) 3-Brom-1-(3-tolyl)-1-(4-biphenylyl)-propin-2-ol vom Schmelzpunkt 131-132 °C.

Aus der DT-OS 2 159 631 ist zwar bereits ein fotografisches Material mit Silberkomplexen bestimmter Propinole, die mit Alkyl- oder Arylresten substituiert sind, bekannt. Diese Verbindungen werden in Kombination mit bestimmten Sensibilisatoren eingesetzt und sollen über eine durch sie bewirkte Verringerung des pAg-Wertes den durch Zugabe des Sensibilisators bewirkten Empfindlichkeitsverlust ausgleichen. Aber durch Zugabe dieser Silberpropinolkomplexe wird der Schleier der fotografischen Materialien deutlich erhöht.

Für die vorliegende Erfindung sind die üblichen Silberhalogenidemulsionen geeignet. Diese können als Silberhalogenid Silberchlorid, Silberbromid oder Gemische davon, eventuell mit einem geringen Gehalt an Silberjodid bis zu 10 Mol-%, enthalten.

Die erfindungsgemäß hergestellten Materialien können mit üblichen Farbentwicklersubstanzen entwickelt werden, z.B.:

N,N-Dimethyl-p-phenylendiamin
4-Amino-3-methyl-N-äthyl-N-methoxyäthylanilin
Monomethyl-p-phenylendiamin
2-Amino-5-diäthylaminotoluol
N-Butyl-N-ω-sulfobutyl-p-phenylendiamin
2-Amino-5-(N-äthyl-N-β-methansulfonamidäthyl-amino)-toluol
N-Äthyl-N-β-hydroxyäthyl-p-phenylendiamin
N,N-Bis-(β-hydroxyäthyl)-p-phenylendiamin
2-Amino-5-(N-äthyl-N-β-hydroxyäthylamino)-toluol und dergleichen.

Weitere brauchbare Farbentwickler sind beispielsweise beschrieben in J. Amer. Chem. Soc. *73*, 3100 (1951).

Das erfindungsgemäß hergestellte fotografische Material kann die üblichen Farbkuppler enthalten, die in der Regel den Silberhalogenidschichten selbst einverleibt sind. So enthält die rotempfindliche

Schicht beispielsweise einen nicht diffundierenden Farbkuppler zur Erzeugung des blaugrünen Teilfarbenbildes, in der Regel einen Kuppler vom Phenol- oder α-Naphtholtyp. Die grünempfindliche Schicht enthält mindestens einen nicht diffundierenden Farbkuppler zur Erzeugung des purpurnen Teilfarbenbildes, wobei üblicherweise Farbkuppler vom Typ des 5-Pyrazolons oder des Indazolons Verwendung finden. Die blauempfindliche Schichteinheit schließlich enthält mindestens einen nicht diffundierenden Farbkuppler zur Erzeugung des gelben Teilfarbenbildes, in der Regel einen Farbkuppler mit einer offenkettigen Ketomethylengruppierung. Farbkuppler dieser Art sind in großer Zahl bekannt und in einer Vielzahl von Patentschriften beschrieben. Beispielhaft sei hier auf die Veröffentlichung « Farbkuppler » von W. PELZ in « Mitteilungen aus den Forschungslaboratorien der Agfa, Leverkusen/München », Band III (1961) und K. VENKATARAMAN in « The Chemistry of Synthetic Dyes », Vol. 4, 341 bis 387 Academic Press, 1971, hingewiesen.

Als weitere nicht diffundierende Farbkuppler können 2 Äquivalentkuppler verwendet werden ; diese enthalten in der Kupplungsstelle einen abspaltbaren Substituenten, so daß sie zur Farbbildung nur zwei Äquivalente Silberhalogenid benötigen im Unterschied zu den üblichen 4-Äquivalentkupplern. Zu den einsetzbaren 2-Äquivalentkupplern gehören beispielsweise die bekannten DIR-Kuppler, bei denen der abspaltbare Rest nach Reaktion mit Farbentwickleroxydationsprodukten als diffundierender Entwicklungsinhibitor in Freiheit gesetzt wird. Weiterhin können zur Verbesserung der Eigenschaften des fotografischen Materials die sogenannten Weißkuppler eingesetzt werden.

Die nicht diffundierenden Farbkuppler und farbgebenden Verbindungen werden den lichtempfindlichen Silberhalogenidemulsionen oder sonstigen Gießlösungen nach üblichen bekannten Methoden zugesetzt. Wenn es sich um wasser- oder alkalilösliche Verbindungen handelt, können sie den Emulsionen in Form von wäßrigen Lösungen, gegebenenfalls unter Zusatz von mit Wasser mischbaren organischen Lösungsmitteln wie Äthanol, Aceton oder Dimethylformamid, zugesetzt werden. Soweit es sich bei den nicht diffundierenden Farbkupplern und farbgebenden Verbindungen um wasser- bzw. alkaliunlösliche Verbindungen handelt, können sie in bekannter Weise emulgiert werden, z.B. indem eine Lösung dieser Verbindungen in einem niedrigsiedenden organischen Lösungsmittel direkt mit der Silberhalogenidemulsion oder zunächst mit einer wäßrigen Gelatinelösung vermischt wird, worauf das organische Lösungsmittel in üblicher Weise entfernt wird. Ein so erhaltenes Gelatineemulgat der jeweiligen Verbindung wird anschließend mit der Silberhalogenidemulsion vermischt. Gegebenenfalls verwendet man zur Einemulgierung derartiger hydrophober Verbindungen zusätzlich noch sogenannte Kupplerlösungsmittel oder Ölformer ; das sind in der Regel höhersiedende organische Verbindungen, die die in den Silberhalogenidemulsionen zu emulgierenden nicht-diffundierenden Farbkuppler und Entwicklungsinhibitor abspaltenden Verbindungen in Form öliger Tröpfchen einschließen. Verwiesen sei in diesem Zusammenhang beispielsweise auf die US-Patentschriften 2 322 027 ; 2 533 514 ; 3 689 271 ; 3 764 336 und 3 765 897.

Als Bindemittel für die fotografischen Schichten wird vorzugsweise Gelatine verwendet. Diese kann jedoch ganz oder teilweise durch andere natürliche oder synthetische Bindemittel ersetzt werden. An natürlichen Bindemitteln sind z.B. Alginsäure und deren Derivate wie Salze, Ester oder Amide, Cellulosederivate wie Carboxymethylcellulose, Alkylcellulose wie Hydroxyäthylcellulose, Stärke oder deren Derivate wie Äther oder Ester oder Carragheenate geeignet. An synthetischen Bindemitteln seien erwähnt Polyvinylalkohol, teilweise verseiftes Polyvinylacetat, Polyvinylpyrrolidon und dergleichen.

Die Emulsionen können auch chemisch sensibilisiert werden, z.B. durch Zusatz schwefelhaltiger Verbindungen bei der chemischen Reifung, beispielsweise Allylisothiocyanat, Allylthioharnstoff, Natriumthiosulfat und ähnliche. Als chemische Sensibilisatoren können ferner auch Reduktionsmittel, z.B. die in den belgischen Patentschriften 493 464 oder 568 687 beschriebenen Zinnverbindungen, ferner Polyamine wie Diäthylentriamin oder Aminomethylsulfinsäurederivate, z.B. gemäß der belgischen Patentschrift 547 323 verwendet werden.

Geeignet als chemische Sensibilisatoren sind auch Edelmetalle bzw. Edelmetallverbindungen wie Gold, Platin, Palladium, Iridium, Ruthenium oder Rhodium. Diese Methode der chemischen Sensibilisierung ist in dem Artikel von R. KOSLOWSKY, Z. wiss. Phot. 46, 65-72, (1951) beschrieben.

Es ist ferner möglich, die Emulsionen mit Polyalkylenoxidderivaten zu sensibilisieren, z.B. mit Polyäthylenoxid eines Molekulargewichtes zwischen 1000 und 20000, ferner mit Kondensationsprodukten von Alkylenoxiden und aliphatischen Alkoholen, Glykolen, cyclischen Dehydratisierungsprodukten von Hexitolen, mit alkylsubstituierten Phenolen, aliphatischen Carbonsäuren, aliphatischen Aminen, aliphatischen Diaminen und Amiden. Die Kondensationsprodukte haben ein Molekulargewicht von mindestens 700, vorzugsweise von mehr als 1000. Zur Erzielung besonderer Effekte kann man diese Sensibilisatoren selbstverständlich kombiniert verwenden, wie in der belgischen Patentschrift 537 273 und in der britischen Patentschrift 727 982 beschrieben.

Die Emulsionen können auch optisch sensibilisiert sein, z.B. mit den üblichen Polymethinfarbstoffen, wie Neutrocyanine, basischen oder sauren Carbocyaninen, Rhodacyaninen, Hemicyaninen, Styrylfarbstoffen, Oxonolen und ähnlichen. Derartige Sensibilisatoren sind in dem Werk von F. M. HAMER « The Cyanine Dyes and related Compounds », (1964) beschrieben.

Die Emulsionen können die üblichen Stabilisatoren enthalten, wie z.B. homöopolare oder salzartige Verbindungen des Quecksilbers mit aromatischen oder heterocyclischen Ringen wie Mercaptotriazole, einfache Quecksilbersalze, Sulfoniumquecksilberdoppelsalze und andere Quecksilberverbindungen. Als

Stabilisatoren sind ferner geeignet Azaindene, vorzugsweise Tetra- oder Pentaazaindene, insbesondere solche. die mit Hydroxyl- oder Aminogruppen substituiert sind. Derartige Verbindungen sind in dem Artikel von BIRR, Z. Wiss. Phot. *47* (1952) 2 bis 58 beschrieben. Weitere geeignete Stabilisatoren sind u.a. heterocyclische Mercaptoverbindungen, z.B. Phenylmercaptotetrazol, quaternäre Benzthiazolderivate, Benzotriazol und ähnliche.

Die Emulsionen können in der üblichen Weise gehärtet sein, beispielsweise mit Formaldehyd oder halogensubstituierten Aldehyden, die eine Carboxylgruppe enthalten, wie Mucobromsäure, Diketonen, Methansulfonsäureester, Dialdehyden und dergleichen.

Weiterhin können die fotografischen Schichten mit Härtern des Epoxityps, des heterocyclischen Äthylenimins oder des Acryloyltyps gehärtet werden. Beispiele derartiger Härter sind z.B. in der deutschen Offenlegungsschrift 2 263 602 oder in der britischen Patentschrift 1 266 655 beschrieben. Weiterhin ist es auch möglich, die Schichten gemäß dem Verfahren der deutschen Offenlegungsschrift 2 218 009 zu härten, um farbfotografische Materialien zu erzielen, die für eine Hochtemperaturverarbeitung geeignet sind.

Es ist ferner möglich, die fotografischen Schichten bzw. die farbfotografischen Mehrschichtenmaterialien mit Härtern der Diazin-, Triazin- oder 1,2-Dihydrochinolin-Reihe zu härten, wie in den britischen Patentschriften 1 193 290, 1 251 091, 1 306 544, 1 266 655, der französischen Patentschrift 7 102 716 oder der deutschen Patentanmeldung P 23 32 317.3 beschrieben ist. Beispiele derartiger Härter sind Alkyl- oder Arylsulfonylgruppen-haltige Diazinderivate, Derivate von hydrierten Diazinen oder Triazinen, wie z.B. 1,3,5-Hexahydrotriazin, Fluor-substituierte Diazinderivate, wie z.B. Fluorpyrimidin, Ester von 2-substituierten 1,2-Dihydrochinolin- oder 1,2-Dihydroisochinolin-N-carbonsäuren. Brauchbar sind weiterhin Vinylsulfonsäurehärter, Carbodiimid- oder Carbamoylhärter, wie z.B. in den deutschen Offenlegungsschriften 2 263 602, 2 225 230 und 1 808 685, der französischen Patentschrift 1 491 807, der deutschen Patentschrift 872 153 und der DDR-Patentschrift 7218 beschrieben. Weitere brauchbare Härter sind beispielsweise in der britischen Patentschrift 1 268 550 beschrieben.

Die erfindungsgemäßen Stabilisierungsmittel werden vorzugsweise den lichtempfindlichen Silberhalogenidemulsionen nach der chemischen Reifung zugesetzt. Selbstverständlich kann man die Stabilisierungsmittel auch anderen fotografischen Schichten zusetzen. Die Konzentration der Stabilisierungsmittel in der Emulsion kann innerhalb weiter Grenzen schwanken. Sie hängt von der Art der Emulsion und dem gewünschten Effekt ab. Im allgemeinen werden mit Mengen von 20 mg bis 3 g, insbesondere von 50-2500 mg pro Mol Silberhalogenid, die gewünschten Effekte erreicht. Bevorzugt sind bei Schwarz-Weiß-Emulsionen Mengen von 50 bis 2500 mg, bei farbkupplerhaltigen Emulsionen Mengen von 200 bis 2500 mg Stabilisierungsmittel pro Mol Silberhalogenid. Die für jede Emulsion optimale Zusatzmenge kann in einfacher Weise durch die üblichen Testversuche ermittelt werden.

Die erfindungsgemäß zu verwendenden Stabilisierungsmittel können auch einem der bei der Verarbeitung fotografischer Materialien verwendeten Bäder zugesetzt werden, beispielsweise einem Stoppbad oder Nachbad. Die Stabilisierungsmittel werden in Bädern in Konzentrationen von 0,4 g/l bis 2 g/l, vorzugsweise von 0,5 g/l bis 1,5 g/l eingesetzt.

Die folgenden Beispiele erläutern die Erfindung, ohne daß diese auf die Ausführungsformen in den Beispielen beschränkt ist.

## Beispiel 1

Eine Silberbromidemulsion mit 14 Mol-% Silberchlorid und 1 Mol-% Silberjodid zur Herstellung eines Kopierpapieres mit harter Gradation wird auf die übliche Weise unter Zusatz von Natriumhexachlororhodinat hergestellt, unter Verwendung von Schwefelsensibilisatoren bis zur optimalen Empfindlichkeit gereift und gewaschen. Das Gelatine-Silber-Verhältnis der fertigen Emulsion beträgt 3,6 : 1, der pH-Wert 5,7.

Die Emulsion wird mit 25 ml pro kg Emulsion einer 0,1-%igen wäßrigen Lösung des optischen Sensibilisators der folgenden Formel sensibilisiert:

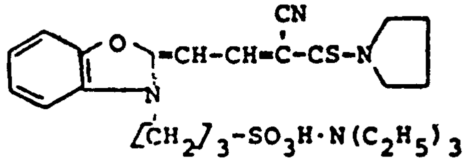

Anschließend wird die sensibilisierte Emulsion in mehrere gleiche Teile geteilt und den einzelnen Proben werden die aus der folgenden Tabelle ersichtlichen Stabilisatoren in den angegebenen Mengen gelöst in Methanol zugesetzt.

Vor dem Vergießen auf einen Schichtträger aus barytiertem Papier erhält jede Probe noch einen Zusatz von 20 ml einer 5 %igen wäßrigen Lösung von Saponin und 0,75 ml einer 30 %igen wäßrigen Lösung von Formaldehyd pro kg Emulsion. Der Silberauftrag berechnet als Silbernitrat beträgt 2,7 g

$AgNO_3/m^2$. Auf die Emulsionsschicht wird noch eine Schutzschicht folgender Zusammensetzung aufgetragen :

20 g Gelatine
1 Liter Wasser
2,4 ml einer 30 %igen wäßrigen Formaldehydlösung
7,5 ml einer 5 %igen wäßrigen Saponinlösung

Die Proben werden frisch und nach 9-monatiger Lagerung bei Raumtemperatur hinter einem $3\sqrt{2}$ Stufenkeil belichtet und in einem Entwickler I der folgenden Zusammensetzung 90 Sekunden bei 20 °C entwickelt. Anschließend wird wie üblich fixiert und gewässert.

Entwickler I

| | |
|---|---|
| p-Methylaminophenol-sulfat | 1 g |
| Natriumsulfit sicc. | 13 g |
| Hydrochinon | 3 g |
| Natriumcarbonat sicc. | 26 g |
| Kaliumbromid | 1 g |

mit Wasser auf 1 l auffüllen.

Wie die in Tabelle 3 zusammengestellten Daten zeigen, wird in den erfindungsgemäßen Materialien eine ausgezeichnete Gradationsstabilisierung und in den meisten Fällen auch Schleierverminderung erreicht.

Weiterhin sind in der Tabelle Werte für den « Langzeitschleier » bei Hochtemperaturverarbeitung angegeben. Diese werden an unbelichteten Materialien bei sonst gleicher Verarbeitung erhalten, welche aber 2 bzw. 4 Minuten bei 30 °C anstatt 90 Sekunden bei 20 °C entwickelt wurden. Aus der Tabelle ist ersichtlich, daß einige der erfindungsgemäßen Materialien einen deutlich geringeren Langzeitschleier aufweisen, als das nicht stabilisierte Material.

Als Vergleichssubstanz A wird ein üblicher Mercaptogruppen enthaltender Stabilisator, nämlich p-3-(2-Mercapto-3,4-dihydro-4-keto-chinazolinyl)-benzolsulfonsäure verwendet. Diese Verbindung ist aus der DT-OS 1 962 605 als Antischleiermittel bekannt und wirkt zwar wie aus Tabelle 3 ersichtlich ist als Antischleiermittel, kann aber nicht die Empfindlichkeit und die Gradation stabilisieren.

BEISPIEL I, TABELLE III

Empfindlichkeit : γ Stufe bei Dichte 1

| | | Frischprüfung | | | | | Nachprüfung nach 9 Monaten | | | | |
|---|---|---|---|---|---|---|---|---|---|---|---|
| Verbindung Nr. | mg/kg | Empfindlichkeit | γ | Schleier | Langzeitschleier 2 Min. | Langzeitschleier 4 Min. | Empfindlichkeit | γ | Schleier | Langzeitschleier 2 Min. | Langzeitschleier 4 Min. |
| Kontroll Probe | — | 16,9 | 5,1 | 0,06 | 0,08 | 0,18 | 19,1 | 3,7 | 0,07 | 0,10 | 0,20 |
| 1 | 131 | 16,0 | 5,2 | 0,06 | 0,07 | 0,10 | 16,0 | 4,9 | 0,07 | 0,07 | 0,12 |
| 3 | 68 | 15,8 | 5,7 | 0,06 | 0,06 | 0,12 | 16,0 | 6,1 | 0,08 | 0,08 | 0,13 |
| 8 | 73 | 16,0 | 4,8 | 0,06 | 0,07 | 0,16 | 15,5 | 5,1 | 0,08 | 0,09 | 0,19 |
| 8 | 147 | 14,5 | 5,6 | 0,06 | 0,07 | 0,16 | 14,9 | 5,7 | 0,07 | 0,09 | 0,18 |
| 12 | 130 | 16,4 | 5,5 | 0,06 | 0,07 | 0,15 | 16,8 | 4,9 | 0,07 | 0,08 | 0,19 |
| 19 | 385 | 16,2 | 4,8 | 0,06 | 0,07 | 0,15 | 16,8 | 4,7 | 0,07 | 0,08 | 0,18 |
| 20 | 430 | 16,3 | 5,3 | 0,04 | 0,07 | 0,14 | 17,3 | 4,8 | 0,05 | 0,08 | 0,18 |
| A | 30 | 16,5 | 5,2 | 0,04 | 0,08 | 0,11 | 18,6 | 3,9 | 0,05 | 0,08 | 0,11 |

Beispiel 2

Eine hochempfindliche Bromjodsilberemulsion mit 5 Mol-% Jodid und einem Gelatine-Silber-Verhältnis von 1, 2 und einem Gehalt von 85 g Silbernitrat pro kg Emulsion wurde mit Schwefel- und Goldverbindungen zur optimalen Empfindlichkeit gereift.

Der Emulsionsansatz wurde in mehrere Teile geteilt und pro kg Emulsion wurden folgende Substanzen zugesetzt:

| | |
|---|---|
| 4-Hydroxy-6-methyl-1,3,3a,7-tetraazainden | 200 mg |
| 1 %ig, wäßrig-alkalische Lösung | |
| Saponin | 600 mg |
| 10 %ig, gelöst in Wasser | |
| Formalinlösung | 10 ml |
| 10 %ig, gelöst in Wasser | |

und die aus der folgenden Tabelle ersichtlichen erfindungsgemäßen Substanzen (1 %ig gelöst in Methanol) in den angegebenen Mengen. Die Mengen wurden so bemessen, daß noch keine stärkere Beeinträchtigung der Empfindlichkeit auftrat.

Die Emulsionen wurden anschließend auf einem Celluloseacetatträger vergossen und getrocknet (Auftrag 7,0 bis 7,2 g berechnet als Silbernitrat pro m²). Die Proben wurden einer Frischprüfung und einer Heizschrankprüfung nach einer Lagerung von 3 Tagen bei 60 °C unterzogen.

Die Proben wurden dann in einem Sensitometer hinter einem Graukeil belichtet und bei 20 °C 16 Minuten lang in einem Entwickler II der folgenden Zusammensetzung entwickelt:

Entwickler II

| | |
|---|---|
| Natriumsulfit sicc. | 50 g |
| Borax | 15 g |
| Hydrochinon | 6 g |
| 1-Phenyl-2-pyrazolidon | 0,5 g |
| Kaliumbromid | 2 g |

mit Wasser auf 1 l auffüllen.

Anschließend folgt ein Stoppbad bestehend aus 10 g Natriumacetat sicc. und 20 g 96 %igem Eisessig in 1 l Wasser. Anschließend wird mit 15 %iger Ammoniumthiosulfat- und 1 %iger Natriumsulfitlösung fixiert und danach gewässert. Die Ergebnisse der sensitometrischen Auswertung sind aus Tabelle 4 ersichtlich.

Die vorteilhafte Wirkung einiger der erfindungsgemäßen Substanzen wird besonders deutlich, wenn das Verhalten des fotografischen Materials bei einer Entwicklung bei einer höheren Temperatur in einem einen Silberkomplexbildner enthaltenden Entwickler in Betracht gezogen wird, der typisch ist für einen Schwarz-weiß-Entwickler für die Umkehrverarbeitung von fotografischen Materialien. Hierzu wird eine unbelichtete Probe 3, 6 und 9 Minuten lang in einem Entwickler III bei 38 °C entwickelt.

Entwickler III

| | |
|---|---|
| 1-Phenyl-3-pyrazolidon | 0,3 g |
| Hydrochinon | 6,0 g |
| Soda sicc. | 35,0 g |
| Natriumsulfit sicc. | 50,0 g |
| Kaliumrhodanid sicc. | 2,5 g |
| Kaliumbromid | 2,0 g |
| Äthylendiamin-N,N,N',N'-tetraessigsäure, $Na_4$-Salz | 2,0 g |

mit Wasser auf 1 l auffüllen und auf pH 10,0 ± 0,1 einstellen.

Die weitere Verarbeitung erfolgt wie oben angegeben. Als Maß für die erhaltene Verschleierung wird die « prozentuale Verschleierung » in Tabelle 4 angegeben; die prozentuale Verschleierung ergibt sich aus dem Verhältnis von entwickeltem Silber (als Silbernitrat) dividiert durch Silber (als Silbernitrat) vor der Verarbeitung und Multiplikation dieses Verhältnisses mit 100. Angegeben ist der Mittelwert, der sich aus den bei den 3 Entwicklungszeiten erhaltenen entwickeltem Silber ergibt.

Aus der Tabelle ist ersichtlich, daß die Substanzen den Schleier, insbesondere den Heizschranksschleier, vermindern und das insbesondere die Substanzen Nr. 3 und 8 den Schleier im Entwickler III vermindern und daher als Antischleiermittel für Umkehrmaterialien geeignet sind.

Als Vergleichssubstanz B wird 3-Mercapto-1,2,4-triazol und als Vergleichssubstanz C 3-Mercapto-5-acetylamino-1-phenyl-1,2,4-triazol die in DT-OS 2 308 530 als Stabilisator beschrieben wird, verwendet. Diese Substanzen können zwar den Schleier bei Verarbeitung in dem Entwickler II im gewissen Umfang vermindern, dabei muß aber bei der Substanz B ein deutlicher Verlust an Empfindlichkeit in Kauf genommen werden. Im Entwickler III zeigen die Vergleichssubstanzen praktisch keine schleiervermindernde Wirkung mehr.

TABELLE IV

| | | Entwickler II | | | | | | Entwickler III |
|---|---|---|---|---|---|---|---|---|
| | | Frischprüfung | | | Heizschrankprüfung | | | proz. Verschleierung Heizschrank-lagerung |
| Verbindung Nr. | mg/kg | Empfind-lichkeit * | γ | Schleier | Empfind-lichkeit * | γ | Schleier | |
| Kontroll-probe | — | | 0,85 | 0,11 | | 0,86 | 0,15 | 34 |
| 3 | 35 | — 1° | 0,85 | 0,10 | — 0,8° | 0,86 | 0,09 | 20 |
| 8 | 367 | — 0,2° | 0,87 | 0,09 | + 0,9° | 0,89 | 0,09 | 21 |
| 9 | 352 | ± 0° | 0,86 | 0,11 | + 0,7° | 0,88 | 0,13 | 27 |
| 12 | 325 | — 1,0° | 0,84 | 0,10 | — 0,5° | 0,86 | 0,12 | 25 |
| 15 | 140 | — 0,4° | 0,86 | 0,11 | — 0,2° | 0,81 | 0,12 | 28 |
| B | 25 | — 2,0° | 0,71 | 0,10 | — 2,5° | 0,68 | 0,13 | 32 |
| C | 56 | ± 0° | 0,75 | 0,10 | — 0,5° | 0,76 | 0,11 | 33 |

* 3° = 1 Blende.

## Beispiel 3

Zu 1 kg einer blau sensibilisierten Bromjodsilber-Emulsion, die 0,24 Mol Silberhalogenid (bestehend aus Silberbromid mit einem Gehalt von 1 Mol-% Silberjodid) enthält, wurden 30 g eines alkalilöslichen Gelbkupplers der Formel

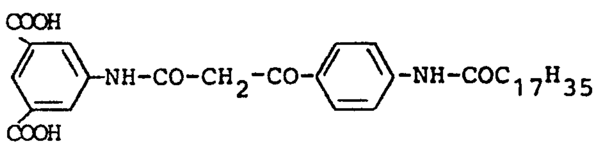

in wäßrig-methanolischer Natronlaugelösung zugefügt. Anschließend wurde der pH-Wert der Emulsion auf 6, 7 eingestellt und 1 g Saponin gelöst in Wasser als Netzmittel, 0,2 g 4-Hydroxy-6-methyl-1,3,3a,7-tetraazainden in wäßrigalkalischer Lösung und 1 g 1,3,5-Triacryl-hexahydro-1,3,5-triazin in methanolischer Lösung als Härtungsmittel zugegeben und die so erhaltene Emulsion in mehrere gleiche Teile geteilt und den einzelnen Proben die aus der folgenden Tabelle ersichtlichen erfindungsgemäßen Verbindungen in den angegebenen Mengen zugesetzt, die so gewählt wurden, daß die Empfindlichkeit möglichst wenig beeinträchtigt wurde.

Die Emulsionsproben wurden auf coronabestrahltes polyäthylenbeschichtetes Papier mit einem Silberauftrag entsprechend 0,75 g $AgNO_3/m^2$ vergossen. Auf die Emulsionsschicht wurde jeweils eine 2 %ige Gelatinelösung, der 0,3 g/l 1,3,5-Triacryl-hexahydro-1,3,5-triazin in methanolischer Lösung zugesetzt worden waren, mit einer Auftragsdicke von 2 g Gelatine/$m^2$ aufgetragen.

Nach Trocknung werden die Materialien hinter einem Stufenkeil belichtet und in dem Entwickler IV der folgenden Zusammensetzung 110 Sekunden bei 35 °C entwickelt.

Entwickler IV

| | |
|---|---|
| Nitrilotriessigsaures Trinatriumsalz | 2 g |
| Natriumsulfit sicc. | 3 g |
| Kaliumbromid | 0,4 g |
| Kaliumcarbonat | 35 g |
| Benzylalkohol | 5 ml |
| Hydroxylaminhydrochlorid | 3 g |
| N-Butyl-N-ω-sulfobutyl-p-phenylendiamin | 6 g |

mit Wasser auf 1 l auffüllen.

Die weitere Verarbeitung umfaßt die folgenden Bäder:

Stoppbad : Pufferlösung aus Natriumacetat und Essigsäure, eingestellt auf pH 6,5.

Bleichfixierbad :

10 g Äthylendiamin-N,N,N',N'-tetraessigsäure, $Na_4$-Salz
2 g Natriumsulfit sicc.,
40 g Natrium-Eisen-(III)-salz der Äthylendiamintetraessigsäure,
13 g Dinatriumphosphat,
100 g Ammoniumthiosulfat,

mit Wasser auf 1 Liter aufgefüllt und eingestellt auf pH 7,2.

Die Verarbeitungszeiten nach der Entwicklung betragen :

| | |
|---|---|
| Stoppbad : | 1 Minute |
| Wässerung : | 1 Minute |
| Bleichfixierung : | 3 Minuten |
| Wässerung : | 3 Minuten |

Man erhält gelbe Bilder des Stufenkeils. Zur Bestimmung des « Langzeitschleiers » werden unbelichtete Proben dem gleichen Verarbeitungsgang unterworfen mit der Abänderung, daß sie 165 Sekunden lang entwickelt werden.

Wie die in der Tabelle 5 zusammengestellten Ergebnisse zeigen, vermindern die Verbindungen den Farbschleier insbesondere bei längerer Entwicklungszeit.

Als Vergleichssubstanz D wird das aus der DT-OS 2 304 321 bekannte Antifarbschleiermittel 2-Amino-5-propin-(2)-ylthio-1,3,4-thiadiazol eingesetzt. Vergleichssubstanz D vermindert den Schleier insbesondere den Langzeitschleier weniger als die erfindungsgemäßen Substanzen und drückt bereits bei relativ geringen Konzentrationen die Empfindlichkeit und verflacht die Gradation.

TABELLE V

| | | | Entwickler IV | | | |
|---|---|---|---|---|---|---|
| Verbindung Nr. | mg/kg | Empfindlichkeit * | Gradation | $D_{Max}$ | Schleier | Langzeitschleier |
| Kontrollprobe | — | 18,5 | 2,54 | 1,75 | 0,22 | 0,28 |
| 1 | 326 | 18,8 | 2,68 | 1,78 | 0,07 | 0,08 |
| 3 | 204 | 18,1 | 2,49 | 1,76 | 0,08 | 0,09 |
| 7 | 324 | 18,6 | 2,48 | 1,77 | 0,13 | 0,11 |
| 8 | 367 | 18,3 | 2,45 | 1,75 | 0,08 | 0,14 |
| 9 | 176 | 18,6 | 2,56 | 1,78 | 0,11 | 0,14 |
| 12 | 325 | 18,0 | 2,51 | 1,73 | 0,18 | 0,16 |
| 14 | 325 | 18,4 | 2,63 | 1,73 | 0,13 | 0,15 |
| 17 | 185 | 17,9 | 2,45 | 1,77 | 0,08 | 0,11 |
| 19 | 385 | 17,9 | 2,58 | 1,75 | 0,10 | 0,16 |
| 21 | 146 | 18,0 | 2,43 | 1,75 | 0,08 | 0,11 |
| 22 | 164 | 18,0 | 2,54 | 1,76 | 0,09 | 0,10 |
| 24 | 446 | 18,6 | 2,53 | 1,78 | 0,12 | 0,13 |
| Vergleichsversuch | | | | | | |
| D | 20 | 18,0 | 2,44 | 1,74 | 0,17 | 0,20 |
| D | 60 | 17,6 | 2,31 | 1,71 | 0,15 | 0,18 |

* Empfindlichkeit = x Stufe bei Dichte 1.

Beispiel 4

Die vorteilhafte Wirkung der erfindungsgemäß zu verwenddenden Substanzen wird besonders deutlich bei einer Entwicklung des fotografischen Materials in Gegenwart von Natriumthiosulfat. Eine

Entwicklung in Gegenwart von Thiosulfat tritt in der Praxis durch Verschleppen von Entwicklersubstanz in das Fixierbad, bzw. in das Bleichfixierbad, insbesondere bei Schnellverarbeitungsverfahren ohne Stoppbad zwischen Entwickler- und Fixier- bzw. Bleichfixierbad, sowie durch Verschleppen von Natriumthiosulfat in den Entwickler, z.B. durch Klammern u.a. Geräteteile auf. Hierbei entsteht bei den üblichen fotografischen Materialien ein hoher Schleier. Wird dagegen erfindungsgemäßes fotografisches Material mit dem thiosulfathaltigen Entwickler V entwickelt, wird dieser Schleier drastisch reduziert, wie aus Tabelle 6 hervorgeht. Als Vergleichssubstanz D wird die gleiche Verbindung wie in Beispiel 3 benutzt. Das Material entspricht dem in Beispiel 3 angegebenen, dem die aus Tab. 6 angegebenen Mengen der erfindungsgemäß zu verwendenden Substanzen zugesetzt sind.

Entwickler V

| | |
|---|---|
| N-Butyl-N-ω-sulfobutyl-p-phenylen-diamin | 5 g |
| Hydroxylaminohydrochlorid | 2,7 g |
| Natriumsulfit sicc. | 3 g |
| Nitrilotriessigsaures Trinatriumsalz | 2 g |
| Kaliumcarbonat | 75 g |
| Kaliumbromid | 1 g |
| Natriumthiosulfat | 1 g |

mit Wasser auf 1 Liter aufgefüllt.

Bei sonst gleicher Verarbeitung wie im Beispiel 3 wird unbelichtetes Material 2 Minuten bei 25 °C im Entwickler V statt im Entwickler IV entwickelt.

TABELLE VI

| Verbindung Nr. | mg/kg | Schleier |
|---|---|---|
| / | / | 0,34 |
| D | 20 | 0,29 |
| D | 60 | 0,27 |
| 1 | 326 | 0,07 |
| 3 | 136 | 0,05 |
| 7 | 324 | 0,02 |
| 8 | 367 | 0,08 |
| 9 | 176 | 0,11 |
| 12 | 325 | 0,04 |
| 14 | 325 | 0,11 |
| 20 | 430 | 0,12 |
| 22 | 164 | 0,14 |
| 24 | 446 | 0,07 |

Die Vergleichssubstanz vermindert den Schleier kaum.

## Beispiel 5

Es wurde ein farbphotographisches Mehrschichtenmaterial A auf einer coronabestrahlten polyäthylenbeschichteten Papierunterlage hergestellt, das eine blausensibilisierte, eine grünsensibilisierte und eine rotsensibilisierte Schicht sowie jeweils zwischen den Emulsionsschichten eine Gelatinetrennschicht enthält, wobei den gießfertigen Emulsionen für den blauempfindlichen Gelbguß sowie den grünempfindlichen Purpurguß pro Mol enthaltenes Silberhalogenid 320 mg der Verbindung 12 in Form einer 1 %igen methanolischen Lösung zugesetzt wurden.

Ein analoges farbphotographisches Mehrschichtenmaterial B wurde als Vergleichsmaterial hersgestellt, wobei die Emulsionen keine der erfindungsgemäßen Verbindungen enthielten.

Das so hergestellte farbphotographische Mehrschichtenmaterial wurde in einem üblichen Sensitometer hinter einem Testbild belichtet, das einen grauen, einen blauen, einen grünen und einen roten Stufenkeil enthält.

Die Entwicklung erfolgte in 110 sec. bei 35 °C in folgendem Entwickler VI

5 g N-Butyl-N-ω-sulfobutyl-p-phenylen-diamin,
1,2 g Hydroxylaminohydrochlorid,
2 g Natriumsulfit sicc.
2 g Natriummetaphosphat,
75 g Kaliumcarbonat,
1 g Kaliumbromid

mit Wasser auf 1 Liter aufgefüllt.

Die weitere Verarbeitung umfaßt die folgenden Bäder:

Stoppbad: eine Pufferlösung aus Natriumacetat und Essigsäure eingestellt auf pH 6,5.

Bleichfixierbad:

10 g Natriumsalz der Äthylendiamintetraessigsäure,
2 g Natriumsulfit sicc.,
40 g Natrium-Eisen-(III)-salz der Äthylendiamintetraessigsäure,
13 g Dinatriumphosphat,
100 g Ammoniumthiosulfat,

mit Wasser auf 1 Liter aufgefüllt und eingestellt auf pH 7,0.

Die Verarbeitungszeiten nach der Entwicklung betragen:

1 Minute Stoppbad,
1 Minute Wässerung,
5 Minuten Bleichfixierbad,
10 Minuten Wässerung.

Nach Belichtung und Verarbeitung ergab der Vergleich der sensitometrischen Auswertung, daß das farbphotographische Mehrschichtenmaterial A, enthaltend die erfindungsgemäße Verbindung, und das Vergleichsmaterial B eine gleiche Empfindlichkeit, eine gleiche Gradation und gleiche Maximaldichte aufwiesen. Den Vergleich der Minimaldichten zeigt die folgende Tabelle:

| Probe | Minimaldichte Gelb | Purpur | Blaugrün |
|---|---|---|---|
| A | 0,14 | 0,12 | 0,10 |
| B | 0,16 | 0,18 | 0,10 |

**Ansprüche**

1. Lichtempfindliches fotografisches Material mit mindestens einer Silberhalogenidemulsionsschicht, gekennzeichnet durch den Gehalt an wenigstens einem Stabilisierungsmittel folgender allgemeinen Formel

$$\mathrm{Hal} - C \equiv C - \overset{\displaystyle R^3}{\underset{\displaystyle R^1}{C}} - R^2$$

worin bedeuten:
$R^1$ ein gegebenenfalls substituierter Alkyl-, Aryl-, Aralkyl- oder heterocyclischer Rest,
$R^2$ OH oder ein stickstoffhaltiger heterocyclischer Rest mit 5 Ringatomen
$R^3$ ein gegebenenfalls substituierter Phenylrest,
Hal Br, J
oder dessen Salz.

2. Lichtempfindliches fotographisches Material nach Anspruch 1, dadurch gekennzeichnet, daß
$R^1$ Alkyl mit 1-4C-Atomen;
Phenyl; Aralkyl; Pyridyl bedeutet und
$R^2$ einem Imidazolyl-(1)-Rest oder einem Rest folgender allgemeinen Formel entspricht

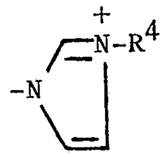

$X^-$

worin

$R^4$ Alkyl, Aralkyl oder Wasserstoff und

X Halogen, Alkylsulfat oder Arylsulfat ist.

3. Lichtempfindliches fotografisches Material nach Anspruch 1, dadurch gekennzeichnet, daß

$R^1$ Alkyl mit 1-4C-Atomen ;

Phenyl ; Aralkyl ; Pyridyl bedeutet und

$R^2$ OH bedeutet.

4. Lichtempfindliches fotografisches Material nach Anspruch 1 bis 3, dadurch gekennzeichnet, daß $R^1$ steht für

Alkyl mit 1 bis 4 C-Atomen,

Phenyl oder mit einer $CF_3O$ ; $CF_3$-, Nitro-,

Phenyl-, Phenoxy- oder Alkylgruppe oder mit Halogen substituiertes Phenyl,

oder für eine Pyridyl-(4)-, Pyridyl-(3)-oder Pyridyl-(2)-Gruppe.

5. Lichtempfindliches fotografisches Material nach Anspruch 1, dadurch gekennzeichnet, daß als Stabilisierungsmittel wenigstens eine Verbindung der folgenden Formel enthalten ist :

a) 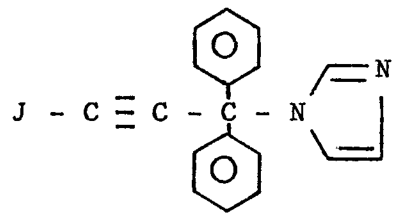

b) 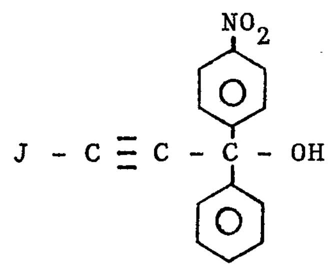

c) 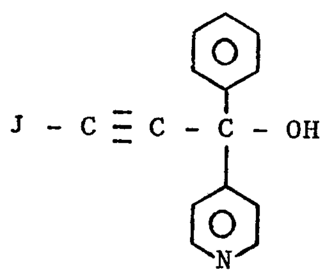

d) 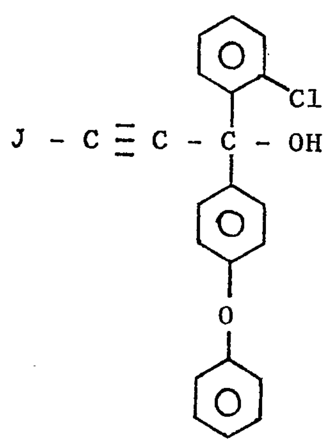

6. Lichtempfindliches fotografisches Material nach Anspruch 1 bis 5, dadurch gekennzeichnet, daß das Stabilisierungsmittel in einer Menge von 20 bis 3 000 mg pro Mol Silberhalogenid enthalten ist.

7. Lichtempfindliches fotografisches Material nach Anspruch 1 bis 6, dadurch gekennzeichnet, daß wenigstens ein Azaindenstabilisator enthalten ist.

8. Verfahren zur Herstellung fotografischer Bilder durch Verarbeitung fotografischer Aufzeichnungsmaterialien, dadurch gekennzeichnet, daß die Verarbeitung in Gegenwart wenigstens einer Verbindung der folgenden Formel oder eines Salzes davon durchgeführt wird :

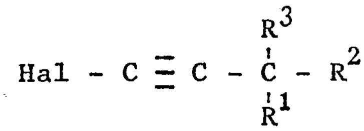

worin bedeuten :

$R^1$ ein gegebenenfalls substituierter Alkyl-, Aryl-, Aralkyl- oder heterocyclischer Rest,
$R^2$ OH oder ein stickstoffhaltiger heterocyclischer Rest mit 5 Ringatomen
$R^3$ ein gegebenenfalls substituierter Phenylrest,
Hal Br, J

oder dessen Salz

9. Bad zur Verarbeitung fotografischer Materialien, gekennzeichnet durch den Gehalt an wenigstens einem Stabilisierungsmittel der folgenden allgemeinen Formel

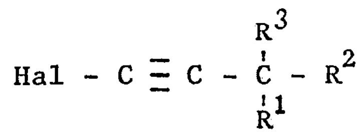

worin bedeuten :

$R^1$ ein gegebenenfalls substituierter Alkyl-, Aryl-, Aralkyl- oder heterocyclischer Rest,
$R^2$ OH oder ein stickstoffhaltiger heterocyclischer Rest mit 5 Ringatomen
$R^3$ ein gegebenenfalls substituierter Phenylrest
Hal Br, J

oder dessen Salz

**Claims**

1. Light sensitive photographic material having at least one silver halide emulsion layer, characterised by containing at least one stabilizer of the following general formula

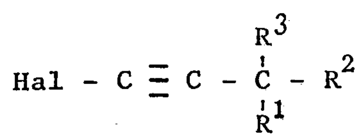

in which

$R^1$ denotes an optionally substituted alkyl, aryl, aralkyl or heterocyclic group,
$R^2$ denotes OH or a nitrogen-containing heterocyclic group having 5 ring atoms,
$R^3$ denotes an optionally substituted phenyl group,
Hal denotes Br or I

or a salt thereof.

2. Light sensitive photographic material according to Claim 1, characterised in that
$R^1$ denotes alkyl with 1 to 4 carbon atoms ; phenyl ; aralkyl ; pyridyl and
$R^2$ represents an imidazolyl-(1)-group or a group of the following general formula

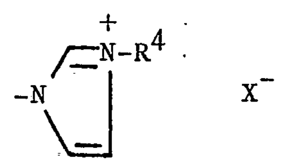

in which

$R_4$ is alkyl, aralkyl or hydrogen and
X is halogen, alkylsulphate or arylsulphate.

3. Light sensitive photographic material according to Claim 1, characterised in that
$R^1$ denotes alkyl with 1 to 4 carbon atoms ; phenyl ; aralkyl ; pyridyl and
$R^2$ denotes hydroxyl.

4. Light sensitive photographic material according to Claim 1 to 3, characterised in that
$R^1$ represents alkyl with 1 to 4 carbon atoms ;
phenyl or phenyl substituted with a $CF_3O$, $CF_3$, nitro, phenyl, phenoxy or alkyl group or with halogen ;
or a pyridyl-(4), pyridyl-(3) or pyridyl-(2) group.

5. Light sensitive photographic material according to Claim 1, characterised in that it contains at least one compound of the following formula as stabilizer :

a) 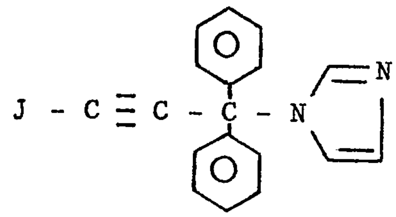

b) 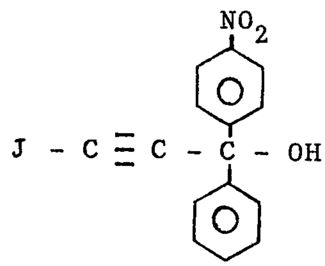

c) 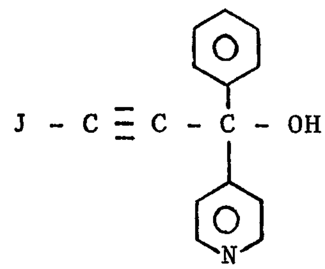

d) 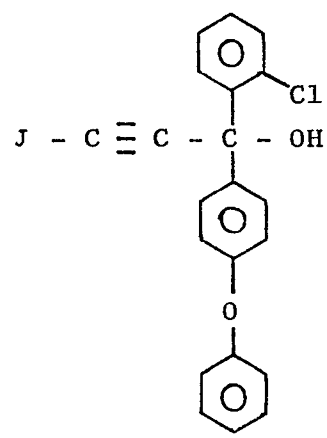

6. Light sensitive photographic material according to Claim 1 to 5, characterised in that the stabilizer is contained in it in a quantity of from 20 to 3,000 mg per mol of silver halide.

7. Light sensitive photographic material according to Claim 1 to 6, characterised in that it contains at least one azaindene stabilizer.

8. Process for the preparation of photographic images by the processing of photographic recording materials, characterised in that processing is carried out in the presence of at least one compound of the following formula or a salt thereof :

$$Hal - C \equiv C - \overset{R^3}{\underset{R^1}{C}} - R^2$$

in which

R¹ denotes an optionally substituted alkyl, aryl, aralkyl or heterocyclic group,

R² denotes hydroxyl or a nitrogen-containing heterocyclic group having 5 ring atoms

R³ denotes an optionally substituted phenyl group,

Hal denotes Br or I

or a salt thereof.

9. Bath for processing photographic materials, characterised by containing at least one stabilizer of the following general formula

$$\mathrm{Hal} - C \equiv C - \overset{\displaystyle R^3}{\underset{\displaystyle R^1}{C}} - R^2$$

in which

$R^1$ denotes an optionally substituted alkyl, aryl, aralkyl or heterocyclic group,

$R^2$ denotes hydroxyl or a nitrogen-containing heterocyclic group having 5 ring atoms,

$R^3$ denotes an optionally substituted phenyl group,

Hal denotes Br or I

or a salt thereof.

**Revendications**

1. Elément photographique photosensible comportant au moins une couche d'émulsion à l'halogénure d'argent, caractérisé en ce qu'il contient au moins un agent stabilisant répondant à la formule générale suivante :

$$\mathrm{Hal} - C \equiv C - \overset{\displaystyle R^3}{\underset{\displaystyle R^1}{C}} - R^2$$

dans laquelle

$R^1$ représente un radical hétérocyclique, un groupe aralkyle, un groupe aryle ou un groupe alkyle éventuellement substitué,

$R^2$ représente OH ou un radical hétérocyclique azoté comportant 5 atomes cycliques,

$R^3$ représente un groupe phényle éventuellement substitué,

Hal représente Br ou I,

ou un sel de ce composé.

2. Elément photographique photosensible suivant la revendication 1, caractérisé en ce que

$R^1$ représente un groupe alkyle contenant 1 à 4 atomes de carbone ; un groupe phényle ; un groupe aralkyle ou un groupe pyridyle et

$R^2$ représente un groupe imidazolyle-(1) ou un radical répondant à la formule générale suivante :

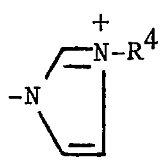

. $X^-$

dans laquelle

$R^4$ représente un groupe alkyle, un groupe aralkyle ou un atome d'hydrogène, et représente un atome d'halogène, un alkylsulfate ou un arylsulfate.

3. Elément photographique photosensible suivant la revendication 1, caractérisé en ce que

$R^1$ représente un groupe alkyle contenant 1 à 4 atomes de carbone ; un groupe phényle ; un groupe aralkyle ou un groupe pyridyle, et

$R^2$ représente OH.

4. Elément photographique photosensible suivant l'une quelconque des revendications 1 à 3, caractérisé en ce que $R^1$ représente un groupe alkyle contenant 1 à 4 atomes de carbone, un groupe phényle ou un groupe phényle substitué par un groupe $CF_3O$, un groupe $CF_3$, un groupe nitro, un groupe phényle, un groupe phénoxy ou un groupe alkyle ou encore par un atome d'halogène, ou également un groupe pyridyle-(4), un groupe pyridyle-(3) ou un groupe pyridyle-(2).

5. Elément photographique photosensible suivant la revendication 1, caractérisé en ce que, comme agent stabilisant, il contient au moins un composé répondant à une des formules suivantes :

a) 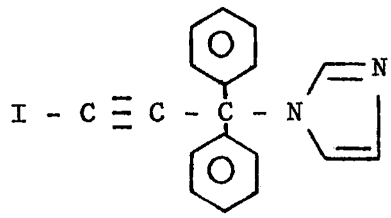

b) 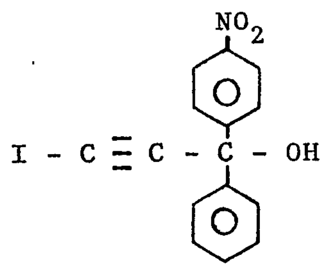

c) 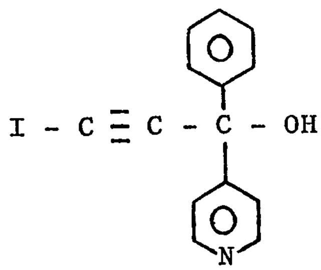

d) 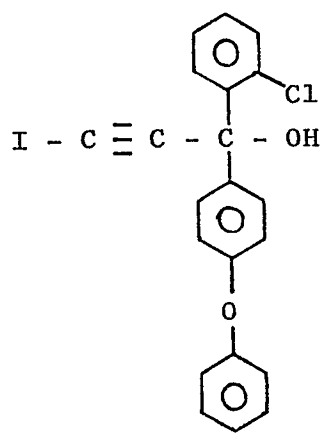

6. Elément photographique photosensible suivant les revendications 1 à 5, caractérisé en ce que l'agent stabilisant est contenu en une quantité de 20 à 3 000 mg par mole d'halogénure d'argent.

7. Elément photographique photosensible suivant les revendications 1 à 6, caractérisé en ce qu'il contient au moins un stabilisant d'azaindène.

8. Procédé de formation d'images photographiques par traitement d'éléments d'enregistrement photographiques, caractérisé en ce qu'on effectue le traitement en présence d'au moins un composé répondant à la formule ci-après ou d'un de ses sels :

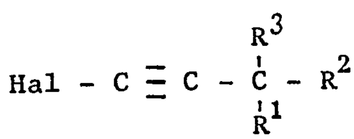

où

$R^1$ représente un radical hétérocyclique, un groupe aralkyle, un groupe aryle ou un groupe alkyle éventuellement substitué,

$R^2$ représente OH ou un radical hétérocyclique azoté comportant 5 atomes cycliques,

$R^3$ représente un groupe phényle éventuellement substitué,

Hal représente Br ou I,

ou un de ses sels.

9. Bain pour le traitement d'éléments photographiques, caractérisé en ce qu'il contient au moins un agent stabilisant répondant à la formule générale suivante :

$$\mathrm{Hal} - C \equiv C - \overset{R^3}{\underset{R^1}{C}} - R^2$$

où

$R^1$ représente un radical hétérocyclique, un groupe aralkyle, un groupe aryle ou un groupe alkyle éventuellement substitué,

$R^2$ représente OH ou un radical hétérocyclique azoté contenant 5 atomes cycliques,

$R^3$ représente un groupe phényle éventuellement substitué,

Hal représente Br ou I,

ou un de ses sels.